# EUROPEAN PATENT SPECIFICATION

(11) **EP 4 166 939 B1**
(45) Date of publication and mention of the grant of the patent: **01.01.2025**
(21) Application number: 21913652.0
(22) Date of filing: 26.11.2021
(51) Int. Cl.: A61B 5/145, A61B 5/00, A61B 5/0531

(54) **METHOD FOR DETECTING SWEAT**
VERFAHREN ZUR DETEKTION VON SCHWEISS
MÉTHODE DE LA DÉTECTION DE LA TRANSPIRATION

(30) Priority: 31.12.2020 CN 202011631972
(43) Date of publication of application: 19.04.2023
(73) Proprietor: Suzhou Institute of Nano-tech and Nano-bionics (SINANO) Chinese Academy of Sciences, Suzhou, Jiangsu 215123 (CN)
(72) Inventor: WANG, Shuqi, Suzhou, Jiangsu 215123 (CN); ZHANG, Ting, Suzhou, Jiangsu 215123 (CN); LIU, Mengyuan, Suzhou, Jiangsu 215123 (CN); LU, Qifeng, Suzhou, Jiangsu 215123 (CN); YANG, Xianqing, Suzhou, Jiangsu 215123 (CN); LI, Lianhui, Suzhou, Jiangsu 215123 (CN)
(74) Representative: Bayramoglu et al.
(86) International application number: PCT/CN2021/133342
(87) International publication number: WO 2022/142910

(56) References cited:
- WO-A1-2018/017619
- WO-A1-2019/060689
- WO-A1-2021/038742
- CN-A- 109 540 984
- CN-A- 110 823 968
- CN-A- 113 640 357
- CN-U- 214 844 940
- US-A1- 2014 012 114
- US-A1- 2014 012 114
- JESSICA FRANCIS ET AL: "Digital nanoliter to milliliter flow rate sensor with in vivo demonstration for continuous sweat rate measurement", LAB ON A CHIP, vol. 19, no. 1, 7 January 2019 (2019-01-07), UK, pages 178 - 185, XP055679527, ISSN: 1473-0197, DOI: 10.1039/C8LC00968F
- JESSICA FRANCIS, ISAAC STAMPER, JASON HEIKENFELD, ELIOT F. GOMEZ: "Digital nanoliter to milliliter flow rate sensor with in vivo demonstration for continuous sweat rate measurement", LAB ON A CHIP, ROYAL SOCIETY OF CHEMISTRY, UK, vol. 19, no. 1, 1 January 2019 (2019-01-01), UK , pages 178 - 185, XP055679527, ISSN: 1473-0197, DOI: 10.1039/C8LC00968F

## Description

### CROSS REFERENCE TO RELATED APPLICATION

This patent application is based on and claims the priority of Chinese Patent Application No. 202011631972.2 filed on December 31, 2020 and entitled "Sweat Sensor and Sweat Sensing System".

### TECHNICAL FIELD

The present disclosure belongs to the technical field of sensors, in particular to a method to detect sweat by a sweat sensor and a method of determining sweat by a sweat sensing system.

### BACKGROUND ART

The abnormal changes of sweat compositions in human body during exercise are related to the blood concentration level, or can directly indicate the health condition of a human body. For example, Na⁺ is the most electrolyte in human sweat, which is the important basis of sweat secretion. The concentration of Na⁺ can reflect different kinds of symptoms of water and salt metabolism disorder in human body. For example, athletes, soldiers, workers, etc. will suffer from hypernatremia due to severe dehydration when working in extreme environments (strenuous exercise, overheated fire rescue, etc.), and the Na⁺ concentration in their sweat and blood is far higher than the normal value. If water and electrolytes are not judged and supplemented timely, it is very likely to cause serious physiological threats or even death.

At present, the traditional sweat sensor cannot simultaneously and continuously detect the sweat volume and the electrolyte concentration in real time, and cannot prevent the mixture of old and new sweat from interfering with the detection of the electrolyte concentration. The patent document US2014/012114A1 discloses methods and apparatus for monitoring of fluid content that are suitable for in-situ, real time and/or continuous monitoring, especially of bodily fluids and in particular the content of sweat. The document also describes fabrication of such an apparatus. The apparatus comprises a multilayer structure comprising at least two electrode layers for detection of fluid content separated by at least one insulating layer. The multilayer structure defines at least one flow channel which provides a flow path for continuous flow of fluid in use, and the electrode layers form part of the sidewall of the flow channel(s). The flow channel(s) may run in a direction substantially perpendicular to the layers. The electrode layers may employ electrochemical detection and may comprise a reference electrode and an ion-sensitive electrode. The article titled "Digital nanoliter to milliliter flow rate sensor with in vivo demonstration for continuous sweat rate measurement" by Jessica Francis, Isaac Stamper, Jason Heikenfeld, Eliot F. Gomez mentions to use digital droplets to measure fluid flow rates and demonstrate their use for sweat rate monitoring, in a device called digital volume dispensing system (DVDS). The DVDS operates by electrically detecting the frequency of droplet generation. The fluid enters the device from the outlet of any pressure-driven channel into the DVDS where a droplet forms in the chamber. The droplet grows until it eventually shorts two electrodes before breaking onto a wick. Since the droplet volume is fixed by the chamber height, the droplet frequency directly measures the flow rate. The patent document WO2018/017619A1 discloses sweat sensing devices configured to periodically measure sweat conductivity and galvanic skin response, devices to measure volumetric sweat flow rate, and devices that combine the three functions.

### SUMMARY

In order to solve the technical problems existing in the prior art, the present disclosure provides a method to detect swear by a sweat sensor and a method of determining sweat by a sweat sensing system which simultaneously and continuously detect the sweat volume and the electrolyte concentration in real time, and can prevent the mixture of old and new sweat from interfering with the detection of the electrolyte concentration.

The invention is defined by method claims 1 and 2.

Compared with the prior art, the present disclosure has the following beneficial effect.

The methods of the present disclosure can simultaneously and continuously detect the sweat volume and the electrolyte concentration in real time, and can prevent the mixture of old and new sweat from interfering with the detection of the electrolyte concentration.

### BRIEF DESCRIPTION OF THE DRAWINGS

The above and other aspects, features and advantages of the embodiments of the present disclosure will become clearer from the following description taken in conjunction with the accompanying drawings, in which:
FIG. 1 is a schematic structural diagram of a sweat sensor according to a first embodiment of the present disclosure.
FIG. 2 is a schematic diagram of the state in which a wearable device with a sweat sensor according to a first embodiment of the present disclosure is placed on the surface of human skin.
FIGS. 3A and 3B are schematic diagrams of the detection principle of a conductance square wave curve and a conductance square wave curve diagram obtained by a sweat sensor under the micro-flow injection pump test according to an embodiment of the present disclosure.
FIG. 4 is a schematic structural diagram of a sweat sensor according to a second embodiment of the present disclosure.
FIG. 5 is a schematic structural diagram of a sweat sensor according to a third embodiment of the present disclosure.
FIG. 6 is a schematic structural diagram of a sweat sensing system according to an embodiment of the present disclosure.

### DETAILED DESCRIPTION OF THE EMBODIMENTS

Hereinafter, specific embodiments of the present disclosure will be described in detail with reference to the drawings. However, the present disclosure can be implemented in many different forms, and the present disclosure should not be construed as limited to the specific embodiments set forth here. On the contrary, these embodiments are provided to explain the principles of the present disclosure and its practical application, so as to enable others skilled in the art to understand the various embodiments of the present disclosure and various modifications suitable for the specific intended application.

As used herein, the term "including" and its variants mean open terms with the meaning of "including but not limited to". The terms such as "based on" and "according to" mean "at least partially based on" and "at least partially according to". The terms "one embodiment" and "an embodiment" mean "at least one embodiment". The term "another embodiment" means "at least one other embodiment". The terms such as "first", "second", etc. can refer to different or identical objects. Other definitions, whether explicit or implicit, can be included below. Unless the context clearly indicates, the definition of a term is consistent throughout the specification.

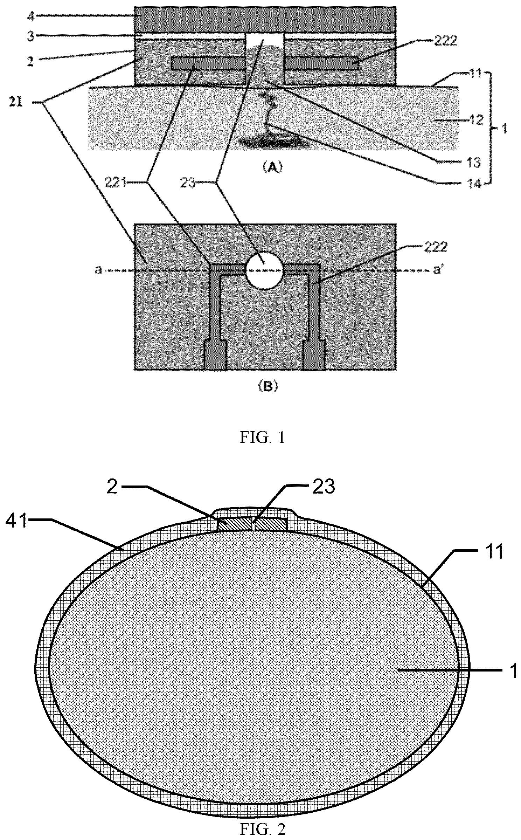

FIG. 1 is a schematic structural diagram of a sweat sensor according to a first embodiment of the present disclosure. In FIG. 1, the diagram (B) shows a top view of a sweat sensor according to a first embodiment of the present disclosure. It is noted that in the diagram (B), in order to clearly show the electrode structure, the adhesive layer 3 and the water-absorbing diffusion layer 4 are not shown. The diagram (A) shows a cross-sectional view of a sweat sensor according to a first embodiment of the present disclosure taken along the line a-a' in the diagram (B). Of course, the diagram (A) additionally shows a human skin system.

As shown in FIG. 1, a sweat sensor according to a first embodiment of the present disclosure comprises a sweat-guiding electrode layer 2, an adhesive layer 3, and a water-absorbing diffusion layer 4.

Specifically, the sweat-guiding electrode layer 2 comprises an insulating layer 21, a conductive electrode (not labeled) provided in the insulating layer 21, and a first through hole (not labeled), wherein the first through hole goes through the insulating layer 21 and the conductive electrode. In one example, the conductive electrode comprises a first electrode 221 and a second electrode 222, wherein the first electrode 221 and the second electrode 222 are located on the same plane, and the central axis of the electrode through hole (not labeled) of the first electrode 221, the central axis of the electrode through hole (not labeled) of the second electrode 222 and the central axis of the first through hole coincide with each other.

In one example, the insulating layer 21 is mainly made of a flexible insulating polymer material, which may be polydimethylsiloxane, silicone rubber, thermoplastic polyester, etc. The thickness of the insulating layer 21 is between 0.1 mm and 2 mm.

The first electrode 221 and the second electrode 222 are embedded inside the insulating layer 21 and located at the middle part in the thickness direction. The first electrode 221 and the second electrode 222 can be thin-film electrodes with certain thickness and width made of carbon nanotubes, graphene, carbon black, carbon fiber, etc., or thin-film electrodes with certain thickness and width made of other materials such as conductive testing metals such as gold, platinum, copper, etc. The thickness of the first electrode 221 and the second electrode 222 is between 0.01 mm and 1 mm, and the width (line width) of the first electrode 221 and the second electrode 222 is smaller than the diameter of each through hole (e.g., an electrode through hole, a first through hole, etc.).

Here, the embedding method of the first electrode 221 and the second electrode 222 in the insulating layer 21 is not particularly limited. For example, in one example, first, an insulating layer 21 is prepared, and the first electrode 221 and the second electrode 222 located on the same level are prepared on the insulating layer 21 using a method such as screen printing. Finally, another insulating layer 21 is prepared on the insulating layer 21, the first electrode 221 and the second electrode 222, and the first electrode 221 and the second electrode 222 are embedded in the position inside the insulating layer 21. In another example, first, an electrode-shaped mold template is prepared using a machining method. The prepolymer, of which the insulating layer 21 is made, is then poured into the mold template using a template replication method, and after the prepolymer is peeled off after being cured and molded, a groove with an electrode shape is formed. The first electrode 221 and the second electrode 222 are filled and prepared in the groove, and then another insulating layer 21 is prepared on the first electrode 221 and the second electrode 222. Finally, the first electrode 221 and the second electrode 222 are embedded in the position inside the insulating layer 21.

At the middle of the insulating layer 21, the first electrode 221 and the second electrode 222, a through hole 23 (consisting of the first through hole, the electrode through hole, etc.) is prepared by a laser cutting method, a template method or a mechanical punching method, and the diameter of the through hole 23 is between 0.5 mm and 2 mm. The effective test surfaces of the first electrode 221 and the second electrode 222 are exposed to the inner wall surface of the through hole 23. Preferably, the cylindrical inner wall of the through hole 23 and the surfaces of the first electrode 221 and the second electrode 222 exposed to the through hole show hydrophobic properties. Therefore, the hydrophobic materials can be selected according to the properties of the materials of the insulating layer 21 and the electrode, and the hydrophobic through hole can also be realized by post-treatment methods, such as silane reagent treatment.

The adhesive layer 3 is provided on the insulating layer 21, and the adhesive layer 3 is provided with a second through hole (not shown) communicated with the first through hole. That is, the through hole 23 goes through the insulating layer 21, the adhesive layer 3, the first electrode 221 and the second electrode 222. The part of the through hole 23 in the insulating layer 21 is set as the first through hole, the part of the through hole 23 in the adhesive layer 3 is set as the second through hole, the part of the through hole 23 in the first electrode 221 is set as the electrode through hole of the first electrode 221, and the part of the through hole 23 in the second electrode 222 is set as the electrode through hole of the second electrode 222.

The adhesive layer 3 is a viscous film fixedly connected between the sweat-guiding electrode layer 2 and the water-absorbing diffusion layer 4, and comprises an ultra-thin double-sided adhesive tape with a fixed thickness (0.01 mm to 0.05 mm in thickness), a prepolymer of viscoelastic polymer, etc. In one example, the adhesive layer 3 prepares a second through hole with the same size as the first through hole at the overlapping position of the first through hole of the sweat-guiding electrode layer 2 by a laser cutting method, a template method or a mechanical punching method, so that sweat flows through the first through hole and the second through hole. Further, the central axis of the first through hole coincides with the central axis of the second through hole.

The water-absorbing diffusion layer 4 is provided on the adhesive layer 3 and covers the second through hole. In one example, the water-absorbing diffusion layer 4 is a film made of a hydrophilic material, including but not limited to water-absorbing materials such as clothing fabrics, paper-based cellulosic films, gels and the like. In this embodiment, the water-absorbing diffusion layer 4 can take clothing itself as the water-absorbing layer, and preferably take breathable sweat-permeable sports tights, wrist guards, palm guards, elbow guards, sweat-absorbing belts and the like as the water-absorbing layer. The thickness of the water-absorbing diffusion layer 4 is not limited. The water-absorbing diffusion layer 4 is integrated with the sweat-guiding electrode layer 2 through the adhesive layer 3 to form a sweat sensor.

FIG. 2 is a schematic diagram of the state in which a wearable device with a sweat sensor according to a first embodiment of the present disclosure is placed on the surface of human skin. As shown in FIG. 2, a sweat sensor according to the first embodiment of the present disclosure is wrapped and fixed by a belt made of elastic fabrics and elastic materials to form a wearable device. Furthermore, the wearable device can be integrated and compatible with sports tights, wrist guards, palm guards, elbow guards, sweat-absorbent belts and other fabrics to form an elastic water-absorbing fixing belt device 41.

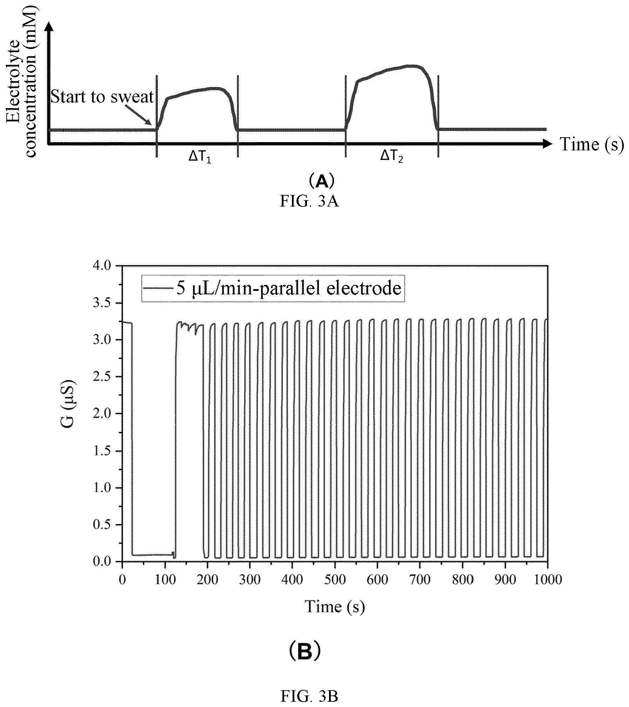

When the sweat sensor according to the embodiment of the present disclosure is provided on the human skin 1, sweat 13 is secreted by sweat glands 14 in the hypodermis 12. When being secreted from sweat glands 14, sweat 13 has a certain pressure up to 70,000 Nm⁻², which is enough to pump sweat 14 into the through hole 23 to be quickly absorbed by the water-absorbing diffusion layer 4. When sweat 13 passes through the inner wall of the through hole 23, the parallel electrodes (i.e., the first electrode 221 and the second electrode 222) exposed to the through hole 23 will record the conductance value of the sweat liquid or the sweat droplet in real time. FIG. 3A is a schematic diagram of the detection principle of a conductance square wave curve, where ΔT₁ represents the duration of a first conductance square wave and ΔT₂ represents the duration of a second conductance square wave. FIG. 3B is a conductance square wave curve diagram obtained by a sweat sensor under the micro-flow injection pump test according to an embodiment of the present disclosure.

As shown in FIG. 3B, the height or amplitude of the conductance square wave curve is correlated with the real-time total sweat electrolyte concentration in the through hole 23, and the sweat volume and sweat rate of sweat droplets passing through the through hole 23 are correlated with the time difference between the conductance square wave curve and the next conductance square wave curve. Therefore, the sweat sensor according to the embodiment of the present disclosure can successfully distinguish the sweat electrolyte concentration from the sweat volume through a real-time continuous conductance square wave curve. At the same time, the sweat sensor has the advantage that the accuracy is not interfered by the mixture of old and new sweat.

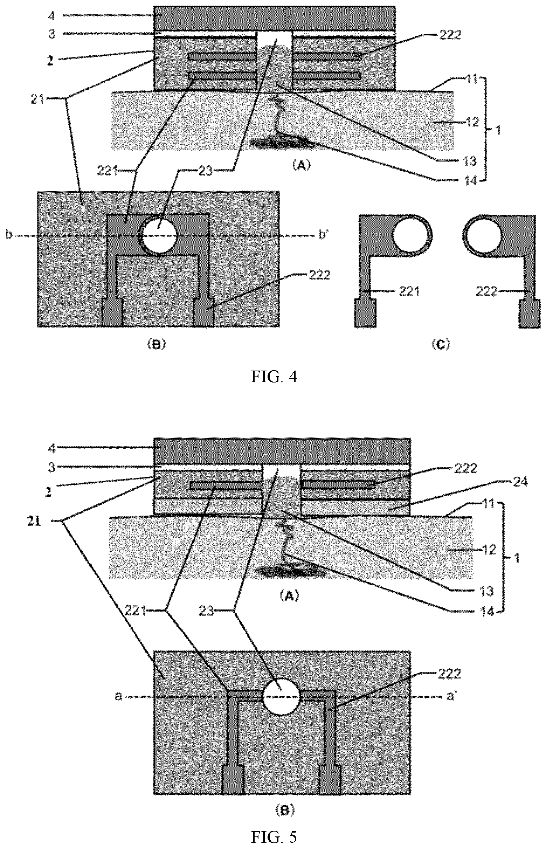

FIG. 4 is a schematic structural diagram of a sweat sensor according to a second embodiment of the present disclosure. In FIG. 4, the diagram (B) shows a top view of a sweat sensor according to a second embodiment of the present disclosure. It is noted that in the diagram (B), in order to clearly show the electrode structure, the adhesive layer 3 and the water-absorbing diffusion layer 4 are not shown. The diagram (A) shows a cross-sectional view of a sweat sensor according to a second embodiment of the present disclosure taken along the line b-b' in the diagram (B). Of course, the diagram (A) additionally shows a human skin system. The diagram (C) shows a schematic structural diagram of the first electrode and the second electrode in the sweat sensor according to the second embodiment of the present disclosure.

As shown in FIG. 4, the structure here is different from the structure of the sweat sensor of the first embodiment shown in FIG. 1 in that the first electrode 221 and the second electrode 222 are not located on the same plane. For example, the second electrode 222 is above the first electrode 221, so that the electrode through hole of the second electrode 222 overlaps with the electrode through hole of the first electrode 221 from top to bottom.

FIG. 5 is a schematic structural diagram of a sweat sensor according to a third embodiment of the present disclosure. In FIG. 5, the diagram (B) shows a top view of a sweat sensor according to a third embodiment of the present disclosure. It is noted that in the diagram (B), in order to clearly show the electrode structure, the adhesive layer 3 and the water-absorbing diffusion layer 4 are not shown. The diagram (A) shows a cross-sectional view of a sweat sensor according to a third embodiment of the present disclosure taken along the line a-a' in the diagram (B). Of course, the diagram (A) additionally shows a human skin system.

As shown in FIG. 5, the structure here is different from the structure of the sweat sensor of the first embodiment shown in FIG. 1 in that the sweat sensor according to the third embodiment of the present disclosure further comprises a contact layer 24, which is provided on the surface of the insulating layer 21 facing away from the adhesive layer 3. The contact layer 24 is provided with a third through hole (not shown) communicated with the first through hole. Further, the central axis of the first through hole coincides with the central axis of the third through hole, that is, the through hole 23 goes through the contact layer 24.

The contact layer 24 has two functions: first, it is convenient to adjust the thickness of the sweat-guiding electrode layer 2; second, when the hardness of the insulating layer 21 is not suitable for direct contact with the skin, the contact layer 24 can achieve good contact and attachment with the skin. The material of the contact layer 24 includes, but is not limited to, polydimethylsiloxane, silicone rubber, thermoplastic polyester, etc. The close bonding between the contact layer 24 and the insulating layer 21 can be achieved by common techniques such as cross-linking and bonding.

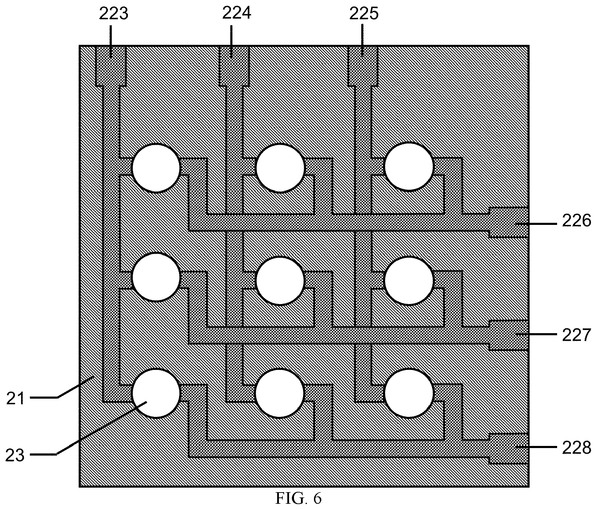

FIG. 6 is a schematic structural diagram of a sweat sensing system according to an embodiment of the present disclosure. For the convenience of description, only conductive electrodes and insulating layers are shown in FIG. 6.

As shown in FIG. 6, the sweat sensing system according to the embodiment of the present disclosure comprises a plurality of sweat sensors according to the second embodiment of the present disclosure shown in FIG. 4. The plurality of sweat sensors are arranged in an array.

In this case, the insulation layer 21, the adhesive layer 3, the water-absorbing diffusion layer 4 and/or the contact layer 24 (if provided) of each sweat sensor are integrated. That is, a plurality of conductive electrodes are provided in an insulating layer 21. An adhesive layer 3 and a water-absorbing diffusion layer 4 are laminated on the insulating layer 21 at a time, and a contact layer 24 is provided on the surface of an insulating layer 21 facing away from an adhesive layer 3.

Of course, each through hole 23 also goes through an adhesive layer 3 to form a plurality of second through holes, and also goes through a contact layer 24 to form a plurality of third through holes. That is, the first through hole (including each electrode through hole), the second through hole and the third through hole are communicated with each other in one-to-one correspondence.

When the plurality of sweat sensors are distributed in an array, the first electrodes of various conductive electrode are located on the same plane, the second electrodes of various conductive electrode are located on the same plane, and the first electrodes and the second electrodes are located on different planes. Therefore, all the first electrodes of the conductive electrodes of each column are connected together and connected to the column conductive terminals, while all the second electrodes of the conductive electrodes of each row are connected together and connected to the row conductive terminals. For example, in FIG. 6, all the first electrodes of a first column of conductive electrodes are connected together and connected to the column conductive terminal 223, all the first electrodes of a second column of conductive electrodes are connected together and connected to the column conductive terminal 224, and all the first electrodes of a third column of conductive electrodes are connected together and connected to the column conductive terminal 225. All second electrodes of a first row of conductive electrodes are connected together and connected to the row conductive terminal 226, all second electrodes of a second row of conductive electrodes are connected together and connected to the row conductive terminal 227, and all second electrodes of a third row of conductive electrodes are connected together and connected to the row conductive terminal 228.

With the sweat sensing system provided above, the data of a plurality of sampling points can be obtained by arranging a plurality of conductive electrodes in an array, so that the accuracy of analysis of sweat volume per unit area and sweat electrolyte concentration can be further improved.

The specific embodiments of the present disclosure have been described above. Other embodiments are within the scope of the appended claims.

The terms "exemplary" and "example" used throughout this specification mean "serving as an example, instance or illustration", rather than mean "preferable to" or "advantageous over" other embodiments. For the purpose of providing an understanding of the described technology, the detailed description comprises specific details. However, these techniques may be practiced without these specific details. In some instances, in order to avoid obscuring the concepts of the described embodiments, well-known structures and devices are shown in the form of a block diagram.

The alternative implementation of the embodiments of the present disclosure according to claims 1 and 2 is described in detail with reference to the drawings hereinabove, but the embodiments of the present disclosure are not limited to the specific details of the above implementation. Within the technical concept of the embodiments of the present disclosure, many simple modifications can be made to the technical solutions of the embodiments of the present disclosure, and these simple modifications all belong to the protection scope of claims 1 and 2 of the present disclosure.

## Claims

1. A method to detect sweat by a sweat sensor, the sweat sensor comprising:
a sweat-guiding electrode layer (2) comprising an insulating layer (21), a conductive electrode provided in the insulating layer (21), and a first through hole, wherein the first through hole goes through the insulating layer (21) and the conductive electrode, and the conductive electrode comprises a first electrode (221) and a second electrode (222);
an adhesive layer (3) provided on the insulating layer (21), wherein the adhesive layer (3) is provided with a second through hole communicated with the first through hole; and
a water-absorbing diffusion layer (4) provided on the adhesive layer (3), wherein the water-absorbing diffusion layer (4) covers the second through hole;
**characterized in that**
conductance values of sweat passing through the first through hole are recorded by the conductive electrode,
a conductance square wave curve is obtained according to the conductance values,
a real-time total sweat electrolyte concentration and a total sweat volume are simultaneously obtained through the conductance square wave curve; and
an amplitude of the conductance square wave curve is correlated with the real-time total sweat electrolyte concentration in the first through hole, and
the total sweat volume and a sweat rate of sweat passing through the first through hole are correlated with a time difference between conductance square waves in the conductance square wave curve.

2. A method of determining sweat by a sweat sensing system, the sweat sensing system comprising:
a sweat-guiding electrode layer (2) comprising an insulating layer (21), a plurality of conductive electrodes provided in the insulating layer (21), and a plurality of first through holes, wherein each of the plurality of first through holes goes through the insulating layer (21) and a corresponding one of the conductive electrodes, and each of the plurality of conductive electrodes comprises a first electrode (221) and a second electrode (222);
an adhesive layer (3) provided on the insulating layer (21), wherein the adhesive layer (3) is provided with a plurality of second through holes, and the plurality of second through holes are communicated with the plurality of first through holes in one-to-one correspondence; and
a water-absorbing diffusion layer (4) provided on the adhesive layer (3), wherein the water-absorbing diffusion layer (4) covers the plurality of second through holes;
**characterized in that**
conductance values of sweat passing through the first through holes are recorded by the conductive electrodes,
a conductance square wave curve is obtained according to the conductance values,
a real-time total sweat electrolyte concentration and a total sweat volume are simultaneously obtained through the conductance square wave curve; and
an amplitude of the conductance square wave curve is correlated with the real-time total sweat electrolyte concentration in the first through holes, and
the total sweat volume and a sweat rate of sweat passing through the first through holes are correlated with a time difference between conductance square waves in the conductance square wave curve.

## Patentansprüche

1. Verfahren zum Erkennen von Schweiß durch einen Schweißsensor, wobei der Schweißsensor Folgendes umfasst:
eine schweißführende Elektrodenschicht (2), umfassend eine Isolationsschicht (21), eine leitende Elektrode, die in der Isolationsschicht (21) bereitgestellt ist, und ein erstes Durchgangsloch, wobei das erste Durchgangsloch durch die Isolationsschicht (21) und die leitende Elektrode führt und die leitende Elektrode eine erste Elektrode (221) und eine zweite Elektrode (222) umfasst;
eine Haftschicht (3), die auf der Isolationsschicht (21) bereitgestellt ist, wobei die Haftschicht (3) mit einem zweiten Durchgangsloch versehen ist, das mit dem ersten Durchgangsloch in Kommunikation gesetzt ist, und
eine wasserabsorbierende Verteilungsschicht (4), die auf der Haftschicht (3) bereitgestellt ist, wobei die wasserabsorbierende Verteilungsschicht (4) das zweite Durchgangsloch bedeckt,
**dadurch gekennzeichnet, dass**
Leitwerte des Schweißes, der durch das erste Durchgangsloch gelangt, von der leitenden Elektrode aufgezeichnet werden;
eine Leitfähigkeitsrechteckwellenkurve je nach den Leitwerten gewonnen wird, eine Echtzeit-Gesamtschweißelektrolytkonzentration und eine Gesamtschweißmenge gleichzeitig durch die Leitfähigkeitsrechteckwellenkurve gewonnen werden und
eine Amplitude der Leitfähigkeitsrechteckwellenkurve mit der Echtzeit-Gesamtschweißelektrolytkonzentration im ersten Durchgangsloch korreliert ist und
die Gesamtschweißmenge und eine Schweißrate des Schweißes, der durch das erste Durchgangsloch gelangt, mit einer Zeitdifferenz zwischen Leitfähigkeitsrechteckwellen in der Leitfähigkeitsrechteckwellenkurve korreliert werden.

2. Verfahren zum Ermitteln von Schweiß durch ein Schweißerfassungssystem, wobei das Schweißerfassungssystem Folgendes umfasst:
eine schweißführende Elektrodenschicht (2), umfassend eine Isolationsschicht (21), eine Vielzahl von leitenden Elektroden, die in der Isolationsschicht (21) bereitgestellt sind, und eine Vielzahl von ersten Durchgangslöchern, wobei ein jedes der Vielzahl von ersten Durchgangslöchern durch die Isolationsschicht (21) und eine entsprechende der leitenden Elektroden führt und eine jede der Vielzahl von leitenden Elektroden eine erste Elektrode (221) und eine zweite Elektrode (222) umfasst;
eine Haftschicht (3), die auf der Isolationsschicht (21) bereitgestellt ist, wobei die Haftschicht (3) mit einer Vielzahl von zweiten Durchgangslöchern versehen ist und die Vielzahl von zweiten Durchgangslöchern mit der Vielzahl von ersten Durchgangslöcher in einer Eins-zu-eins-Entsprechung in Kommunikation gesetzt ist, und
eine wasserabsorbierende Verteilungsschicht (4), die auf der Haftschicht (3) bereitgestellt ist, wobei die wasserabsorbierende Verteilungsschicht (4) die Vielzahl von zweiten Durchgangslöchern bedeckt,
**dadurch gekennzeichnet, dass**
Leitwerte des Schweißes, der durch die ersten Durchgangslöcher gelangt, von den leitenden Elektroden aufgezeichnet werden;
eine Leitfähigkeitsrechteckwellenkurve je nach den Leitwerten gewonnen wird;
eine Echtzeit-Gesamtschweißelektrolytkonzentration und eine Gesamtschweißmenge gleichzeitig durch die Leitfähigkeitsrechteckwellenkurve gewonnen werden und
eine Amplitude der Leitfähigkeitsrechteckwellenkurve mit der Echtzeit-Gesamtschweißelektrolytkonzentration in den ersten Durchgangslöchern korreliert ist und
die Gesamtschweißmenge und eine Schweißrate des Schweißes, der durch die ersten Durchgangslöcher gelangt, mit einer Zeitdifferenz zwischen Leitfähigkeitsrechteckwellen in der Leitfähigkeitsrechteckwellenkurve korreliert werden.

## Revendications

1. Procédé de détection de transpiration à l'aide d'un capteur de transpiration, le capteur de transpiration comprenant :
une couche d'électrode de guidage de sueur (2) comprenant une couche isolante (21), une électrode conductrice prévue dans la couche isolante (21) et un premier trou traversant, dans lequel le premier trou traversant s'étend à travers la couche isolante (21) et l'électrode conductrice, et l'électrode conductrice comprend une première électrode (221) et une deuxième électrode (222) ;
une couche adhésive (3) prévue sur la couche isolante (21), dans lequel la couche adhésive (3) est pourvue d'un deuxième trou traversant en communication avec le premier trou traversant ; et
une couche de diffusion absorbant l'eau (4) prévue sur la couche adhésive (3), dans lequel la couche de diffusion absorbant l'eau (4) recouvre le deuxième trou traversant ;
**caractérisé en ce que**
des valeurs de conductance de sueur passant à travers le premier trou traversant sont enregistrées par l'électrode conductrice,
une courbe d'onde carrée de conductance est obtenue en fonction des valeurs de conductance,
une concentration totale en électrolyte de sueur en temps réel et un volume total de sueur sont obtenus simultanément à travers la courbe d'onde carrée de conductance ; et
une amplitude de la courbe d'onde carrée de conductance est corrélée avec la concentration totale en électrolyte de sueur en temps réel dans le premier trou traversant, et
le volume total de sueur et le taux de transpiration de sueur passant à travers le premier trou traversant sont corrélés avec la différence temporelle entre les ondes carrées de conductance dans la courbe d'onde carrée de conductance.

2. Procédé de détermination de transpiration à l'aide d'un système capteur de transpiration, le système capteur de transpiration comprenant :
une couche d'électrode de guidage de sueur (2) comprenant une couche isolante (21), une pluralité d'électrodes conductrices prévues dans la couche isolante (21) et une pluralité de premiers trous traversants, dans lequel chacun de la pluralité de premiers trous traversants s'étend à travers la couche isolante (21) et une électrode correspondante parmi les électrodes conductrices, et chacune de la pluralité des électrodes conductrices comprend une première électrode (221) et une deuxième électrode (222) ;
une couche adhésive (3) prévue sur la couche isolante (21), dans lequel la couche adhésive (3) est pourvue d'une pluralité de deuxièmes trous traversants, et la pluralité de deuxièmes trous traversants sont en communication avec la pluralité de premiers trous traversants en vis-à-vis un à un ; et
une couche de diffusion absorbant l'eau (4) prévue sur la couche adhésive (3), dans lequel la couche de diffusion absorbant l'eau (4) recouvre la pluralité de deuxièmes trous traversants ;
**caractérisé en ce que**
des valeurs de conductance de sueur passant à travers les premiers trous traversants sont enregistrées par les électrodes conductrices,
une courbe d'onde carrée de conductance est obtenue en fonction des valeurs de conductance,
une concentration totale en électrolyte de sueur en temps réel et un volume total de sueur sont obtenus simultanément à travers la courbe d'onde carrée de conductance ; et
une amplitude de la courbe d'onde carrée de conductance est corrélée avec la concentration totale en électrolyte de sueur en temps réel dans les premiers trous traversants, et
le volume total de sueur et le taux de transpiration de sueur passant à travers les premiers trous traversants sont corrélés avec la différence temporelle entre les ondes carrées de conductance dans la courbe d'onde carrée de conductance.
